# EUROPÄISCHE PATENTANMELDUNG

(11) **EP 1 913 900 A1**
(43) Veröffentlichungstag der Anmeldung: **23.04.2008**
(21) Anmeldenummer: 08000614.1
(22) Anmeldetag: 29.06.2004
(51) Int. Cl.: A61F 2/24

(54) **Ringprothese für Anuloplastie**

(62) Teilanmeldung aus: 04015190.4
(71) Anmelder: Sievers, Hans-Hinrich, Prof. Dr. med., 24119 Kronshagen (DE)
(72) Erfinder: Sievers, Hans-Hinrich, Prof. Dr. med., 24119 Kronshagen (DE)
(74) Vertreter: Castell, Klaus

(57) **Zusammenfassung**

Das Implantat weist einen ringförmig gebogenen Grundkörper (2) auf, wobei die vom Grundkörper umgebene gedachte Fläche gebogen ist. Vorzugsweise ist die vom Grundkörper umgebene Fläche unsymmetrisch. Besonders vorteilhaft ist es, wenn der Grundkörper von einem festen Material umgeben ist und dieses von einem relativ weichen Material umgeben ist. Dies bietet die Möglichkeit im relativ festen Material Fixierungslaschen (12) auszubilden. Das Implantat weist Stichkanäle (25) auf.

Derartige Implantate eignen sich vor allem als Herzklappenring.

## Beschreibung

Die Erfindung betrifft ein Implantat mit einem ringförmigen Grundkörper.

Implantate mit ringförmigen Grundkörpern werden an unterschiedlichsten Stellen des Körpers verwendet. Hierbei sind insbesondere Ein-und Ausgänge von Gefäßen, künstliche Darmausgänge und ähnliches zu nennen. Die Erfindung betrifft insbesondere Stabilisierungsringe für einen Herzklappenanulus.

Die Herzklappen am menschlichen oder tierischen Herzen haben die Aufgabe, je nach Kontraktionszustand den Blutfluss im geöffneten Zustand zuzulassen bzw. im geschlossenen Zustand einen Rückfluss zu verhindern. Diese Funktion wird durch die Herzklappensegel, die im geschlossenen Zustand der Klappen passgenau schließen, geregelt. Die gesamte Geometrie der Herzklappen stellt sicher, dass die Segel präzise übereinander liegen, um einen Rückfluss zu verhindern.

Verschiedene Erkrankungen am Herzen können zu einer Verformung der Herzklappenanuli führen. Hierdurch kann es zur Verformung der gesamten Klappengeometrie kommen. Dies führt dazu, dass die Klappensegel nicht mehr präzise aufeinander liegen und dadurch die Funktion der Herzklappe eingeschränkt ist. Dies geht häufig mit einer Leckage durch die geschlossene Herzklappe bzw. einer Verschlechterung der Klappendynamik und damit einer Verzögerung der Verschlussphase einher. Die Leistungsfähigkeit des Herzens verschlechtert sich dadurch wesentlich.

Derart geschädigte Klappen können durch künstliche Herzklappenprothesen ersetzt werden. Die Prothesen erfüllen die Funktion der geschädigten Klappe zwar hinreichend; durch die eingeschränkte Dauerfestigkeit bzw. körperfremde Werkstoffe weist diese Methode jedoch gewisse Limitationen auf.

Eine aussichtsreiche Alternative zur künstlichen Herzklappenprothese stellt die chirurgische Korrektur des Klappenanulus dar. Hierbei wird ein mehr oder weniger flexibler Ring im Klappenanulus befestigt, der die physiologische Geometrie wieder herstellen soll.

Diese Verfahren basieren auf Erfindungen von Carpentier (US 3,656,185) und Cooley (US 4,164,046) und sind klinisch etabliert.

Die verwendeten Ringe sind jedoch sowohl als starre als auch als flexible Ringe in vielen Fällen nicht optimal dazu geeignet, auf einfache Art und Weise eine Formgebung des Klappenanulus zu bewirken, die ein dichtes Schließen der Klappensegel sicherstellt.

Der Erfindung liegt daher die Aufgabe zugrunde, ein Implantat mit einem ringförmigen Grundkörper so weiter zu entwickeln, dass es für viele Einsatzbereiche möglichst optimal passt.

Diese Aufgabe wird mit einem Implantat mit einem ringförmigen Grundkörper gelöst, bei dem die vom Grundkörper umgebene gedachte Fläche in einem ersten Quadranten nach innen gebogen ist und in einem daran anschließenden zweiten Quadranten zunächst nach innen und dann außen gebogen ist.

Das erfindungsgemäße Implantat hat einen ringförmigen Grundkörper, der so ausgebildet ist, dass die vom Grundkörper umgebene gedachte Fläche nach besonderen Vorgaben gebogen ist. Dies ermöglicht es Ringe herzustellen, die optimal an die physiologischen Gegebenheiten angepasst sind und auch im mechanisch unbelasteten Zustand in dieser Form verbleiben. Somit können je nach Anwendungsfall unterschiedlichste Ringformen gefertigt werden, die entweder individuell an den kranken Gefäßausgang angepasst sind oder je nach Größe und Biegung für unterschiedliche Einsatzzwecke vorgefertigt und verkauft werden können.

Vorteilhaft ist es, wenn die vom Grundkörper umgebene gedachte Fläche in einem dritten und in einem vierten Quadranten nach außen gebogen ist. Es hat sich herausgestellt, dass für den medizinischen Einsatz eine etwa vollständig im ersten Quadranten liegende konkave Umfangslinie vorteilhaft ist. Daran schließen an einer Seite über die zwei folgenden Quadranten konvexe Umfangslinien an und der vierte Quadrant ist etwa zur Hälfte konvex und in der weiteren Hälfte abgeflacht oder sogar konkav ausgebildet.

Für den klinischen Einsatz ist es besonders vorteilhaft, wenn die vom Grundkörper umgebene gedachte Fläche in sich gebogen ist.

Bekannte Implantatringe weisen eine zweidimensionale Geometrie auf. Zum Teil lassen sich diese Ringe auch in der dritten Dimension verformen. Hierzu sind die Ringe elastisch ausgebildet. Dies führt jedoch dazu, dass der Ring im nicht mechanisch belasteten Zustand wieder eine zweidimensionale Struktur einnimmt. Die in sich gebogene Form des Grundkörpers führt zu einer Auslieferung des Implantats mit einer vorgegebenen Formgebung, die während der Operation nicht mehr oder nur noch unwesentlich angepasst werden muss.

Eine vorteilhafte Ausführungsform sieht vor, dass die vom Grundkörper umgebene gedachte Fläche im Querschnitt einen Wendepunkt aufweist. Von der Seite betrachtet hat ein derartiger Grundkörper konkave und konvexe Bereiche und je nach Anwendungsfall können sogar mehrere Wendepunkte vorliegen. Dies erlaubt es optimale Ringformen zu gestalten, die sowohl in der Draufsicht als auch in der Seitenansicht von einfachen geometrischen Grundformen abweichen. Die vom Grundkörper umgebene gedachte Fläche hat somit zumindest in einer Schnittlinie eine Sattelform oder eine S-förmige Biegung.

Es hat sich herausgestellt, dass die Form des Ringes nicht unbedingt eine Kreisform sein muss. Vorteilhaft ist es, wenn die vom Grundkörper umgebene gedachte Fläche unsymmetrisch ist. Insbesondere ringförmige Grundkörper, die weder punkt- noch spiegelsymmetrisch aufgebaut sind, ermöglichen eine optimale Anpassung an die im Körper vorzufindende Anulusgeometrie. Dies ist vor allem für Implantate im Bereich des Herzklappenanulus von Bedeutung.

Eine Steigerung der Anpassungsfähigkeit - aber möglicherweise auch eine Reduzierung der Haltbarkeit - wird dadurch erzielt, dass der Grundkörper plastisch verformbar ist. Dies ermöglicht es den Grundkörper noch kurz vor der Operation nachzuformen, so dass er bestmöglich an die vorliegenden physiologischen Gegebenheiten anpassbar ist.

Besonders bevorzugt ist ein Material, das durch eine physikalische oder chemische Behandlung für einen bestimmten Zeitraum plastisch verformbar gemacht werden kann. Dies ermöglicht es, vor der Implantation den Ring optimal zu formen, wobei der Ring danach und insbesondere in der implantierten Situation nicht mehr verformbar ist oder nur noch elastisch verformbar ist. Dieses Material kann beispielsweise durch eine Wärmebehandlung plastisch verformbar gemacht werden. Sowie das Material auf die Körpertemperatur unter 42 °C abkühlt, verliert es seine plastische Verformbarkeit.

Darüber hinaus können auch Kunststoffe mit Memory-Effekt Verwendung finden. Derartige Kunststoffmaterialien können beliebig verformt werden. Sowie sie jedoch in einem Temperaturbereich der menschlichen Körpertemperatur gebracht werden, nehmen sie eine vorher definierte Form ein.

Vor allem für den Einsatz ringförmiger Grundkörper zur Verstärkung des Herzklappenanulus ist es vorteilhaft, wenn die vom Grundkörper umgebene gedachte Fläche mindestens einen konkav gebogenen Randbereich aufweist. Dieser konkav gebogene Randbereich ist im Fall der Behandlung eines Herzklappenanulus vorteilhaft zwischen den Trigoni Fibrosae angeordnet.

Eine Ausführungsform sieht vor, dass der Grundkörper von einem mehrschichtigen Material, vorzugsweise einem Gewebe, umgeben ist. Diese Ausführungsform ist unabhängig von den zuvor beschriebenen Ringgeometrien vorteilhaft und erfindungswesentlich. Beim Einsatz der bekannten Implantate mit ringförmigen Grundkörpern kann es aufgrund der starken Belastung durch die hohen Lastwechsel zu Problemen der Verankerung am umgebenden Gewebe, wie beispielsweise des Herzens, kommen. Bekannte Implantate weisen einen metallischen Ring auf, der von einem Nahtring umgeben ist. Hierbei entsteht das Problem, dass es zur Ablösung der Struktur des Nahtrings vom metallischen Kern kommen kann. Außerdem kann es durch die punktuelle Belastung des Nahtrings zur Zerstörung der Struktur und zum Ausriss einzelner Fasern kommen.

Dem wird erfindungsgemäß dadurch begegnet, dass der Grundkörper von einem mehrschichtigen Gewebe umgeben ist. Vorzugsweise ist der Grundkörper von einem festen Material umgeben und dieses ist von einem relativ weichen Material umgeben. Dadurch entsteht zumindest eine Dreischichtigkeit aus Grundkörpermaterial, dem den Grundkörper umgebenden festen Material und dem dieses Material umgebenden relativ weichen Material.

Hierbei wird der Grundkörper vorzugsweise von einem metallischen Material gebildet. Wie oben erläutert sind jedoch auch verschiedene Kunststoffmaterialien einsetzbar.

Der mehrschichtige Aufbau erlaubt es ein Kernmaterial zu verwenden, das wesentlich zur Formgebung beiträgt. Die darüber angeordnete erste Schicht stellt eine gute Verbindung zum Kernmaterial her und besteht aus einem relativ festen Material. Die zweite Schicht tritt im implantierten Zustand mit dem umgebenden Gewebe in Kontakt und weist die hierzu übliche weiche Beschaffenheit auf. Bei der Fixierung des Ringes kann die Naht wie üblich durch die zweite Schicht geführt werden. Zur festeren Verankerung kann die Naht jedoch auch durch das innere, festere Material geführt werden. Dadurch wird beispielsweise eine wesentlich verbesserte Fixierung eines Klappenringes auch bei hohen punktuellen Belastungen gewährleistet.

Eine weitere Ausführungsform sieht vor, dass der Grundkörper Stichkanäle aufweist. Die Befestigung des Implantats am umliegenden Gewebe kann entweder durch Annähen an einem den Grundkörper umgebenden Gewebe erfolgen oder der Grundkörper besteht aus weicherem Material, das mit der Nadel durchstochen werden kann. Besonders vorteilhaft ist es jedoch, wenn im Grundkörper Stichkanäle schon vorgefertigt angeordnet sind, die während der Implantation der Verankerung der Fäden dienen können.

Je nach Anwendungsfall wird vorgeschlagen, dass die Stichkanäle in der Draufsicht rund oder oval ausgebildet sind.

Um die Stichkanäle leichter aufzufinden und um die Nadel durch die Stichkanäle zuführen, wird vorgeschlagen, dass die Stichkanäle trichterförmige Öffnungen aufweisen. Die Stichkanäle sind somit im Randbereich aufgeweitet. Dies vergrößert die Öffnung, während im mittleren Bereich eine kleinere Stichkanalöffnung die Stabilität des Implantats gewährleistet.

Eine vorteilhafte Ausführungsform sieht vor, dass die Stichkanäle in der Mitte des Grundkörperquerschnitts angeordnet sind. Hierdurch wird die Stabilität des Implantats am wenigsten eingeschränkt. Je nach Anwendungsfall ist es jedoch auch vorteilhaft, die Stichkanäle versetzt zur Mitte des Grundkörperquerschnitts, vorzugsweise nach außen verlagert, anzuordnen. Hierdurch wird die Naht in den äußeren Bereich des Implantats verlegt. Je nach chirurgischer Prämisse können derart verschiedene Positionen der Naht relativ zum Implantatkörper festgelegt werden. Beispielsweise kann der Stichkanal auch versetzt zur vom Grundkörper umgebenen gedachten Fläche angeordnet werden.

Die Stichkanäle erlauben es, je nach Anwendungsfall sogar vollständig auf ein, den Grundkörper umgebendes Gewebes zu verzichten. Die Stichkanäle können jedoch auch in Kombination mit einem den Grundkörper umgebenden Gewebe unterschiedliche Befestigungsmöglichkeiten des Implantats während der Operation gewährleisten.

Weiterhin ist es vorteilhaft, wenn das Implantat eine Fixierungslasche aufweist. Eine derartige Fixierungslasche ist auch unabhängig von der Geometrie und dem Schichtaufbau des Implantats vorteilhaft und erfindungswesentlich. Eine Fixierungslasche vergrößert den geometrischen Bereich, in dem bei der Implantation der Faden fixiert werden kann. Ein Ausführungsbeispiel sieht vor, dass die Fixierungslasche in einem den Grundkörper umgebenden Material angeordnet ist. Da der Grundkörper in der Regel ein metallisches Material ist, ermöglicht das Anordnen der Fixierungslasche in einem den Grundkörper umgebenden Material eine besonders einfache Herstellung des Implantats.

Hierbei kann das dem Grundkörper umgebende Material, in dem die Fixierungslasche ausgebildet ist, ein festes Material sein.

Unter festem Material wird im Rahmen der Erfindungsbeschreibung ein Material verstanden, das es erlaubt, mit einer Nadel einen Faden durch dieses Material zu ziehen, um das Material mit dem das Implantat umgebenden Gewebe zu verbinden. Andererseits soll das feste Material jedoch eine höhere Festigkeit aufweisen als bekannte Gewebematerialien, die dazu verwendet werden, metallische Grundkörper im Körper durch Nähen zu verankern.

Die Fixierungslasche oder mehrere Fixierungslaschen können an den besonders belasteten Bereichen des Implantats vorgesehen werden, um eine sichere Verankerung zu gewährleisten. Die Implantation wird jedoch dadurch erleichtert, dass die Fixierungslasche den Grundkörper ringförmig umgibt. Dies ermöglicht es das Implantat auf der gesamten Umfangslinie nicht nur mit dem weicheren Gewebematerial zu verankern sondern die Naht durch die Fixierungslasche aus vorzugsweise festerem Material zu führen.

Insbesondere für die Behandlung eines verformten Herzklappenanulus ist es von Vorteil, wenn das Implantat mindestens einen verstärkt ausgebildeten Fixationspunkt aufweist. Auch dieses Merkmal des erfindungsgemäßen Implantats ist auch ohne die vorher beschriebenen Merkmale erfindungswesentlich. Vorzugsweise werden an zwei Seiten eines konkav gebogenen Randbereichs Fixationspunkte angeordnet. Bei einer Herzklappenanulusverstärkung sind diese Fixationspunkte so ausgeführt, dass hier zentral die Last in den Klappenring eingekoppelt werden kann. Die Position der Fixationspunkte ist in diesem Fall vorteilhafter Weise so gewählt, dass sie an die anatomischen Gegebenheiten angepasst eine Fixierung an den Trigoni Fibrosae zulässt.

Vorteilhafte Ausführungsbeispiele eines erfindungsgemäßen Implantats sind in der Zeichnung dargestellt und werden im Folgenden näher erläutert.
Es zeigt
- Figur 1: eine Draufsicht auf ein ringförmiges Implantat,
- Figur 2: eine Seitenansicht von vorne auf das in Figur 1 gezeigte ringförmige Implantat,
- Figur 3: eine Seitenansicht von der Seite auf das in Figur 1 gezeigte ringförmige Implantat,
- Figur 4: einen Schnitt durch einen Ringbereich des in Figur 1 gezeigten Implantats,
- Figur 5: einen Schnitt durch einen Ringbereich des in Figur 1 gezeigten Implantats im Bereich einer Fixierungslasche,
- Figur 6: eine Draufsicht auf ein Implantat entsprechend dem in Figur 1 gezeigten Implantat jedoch mit zwei Fixationspunkten,
- Figur 7: eine Draufsicht auf ein Implantat mit eingezeichneten Quadranten und Stichkanälen,
- Figur 8: ein Schnitt durch das in Figur 7 gezeigte Implantat,
- Figur 9: eine Ansicht des in Figur 8 gezeigten Schnitts mit einem umhüllenden Gewebe,
- Figur 10: eine alternative Ausführungsform im Schnitt durch das Implantat mit dezentralen Stichkanälen,
- Figur 11: eine weitere Ausführungsform in einer Schnittdarstellung mit einem Stichkanal, der versetzt zur vom Grundkörper umgebenen gedachten Fläche angeordnet ist,
- Figur 12: eine vergrößerte Aufsicht auf einen Ausschnitt eines Grundkörpers mit rund ausgebildeten Stichkanälen und
- Figur 13: eine vergrößerte Aufsicht auf einen Ausschnitt eines Grundkörper mit oval ausgebildeten Stichkanälen.

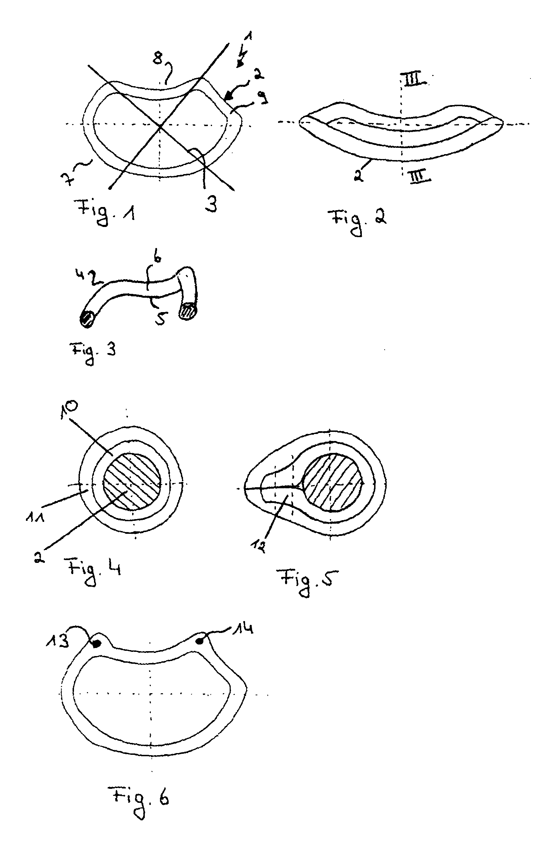

Das in Figur 1 gezeigte Implantat 1 hat einen ringförmigen Grundkörper 2, der eine gedachte Fläche 3 umgibt. Die in den Figuren 2 und 3 gezeigten Seitenansichten erläutern, dass die gedachte Fläche 3 eine gebogene Form aufweist. Im vorliegenden Fall ist sie s-förmig gebogen. Sie hat somit gegenläufig gebogene Bereiche 4 und 5, zwischen denen ein Wendepunkt 6 liegt.

Insbesondere die Figur 1 zeigt, dass die vom Grundkörper umgebene gedachte Fläche unsymmetrisch ist. Der Bereich 7 des Grundkörpers 2 hat etwa die Form einer Ellipse während der restliche Umfangsbereich im Bereich 8 gegenläufig gebogen ist und im Bereich 9 etwa gradlinig ausgebildet ist.

Die Figur 4 zeigt, dass der Grundkörper 2 von zwei Materialschichten 10 und 11 umgeben ist. In diesem Ausführungsbeispiel besteht der Grundkörper aus einem metallischen Kernmaterial. Dieses ist von einem relativ festen Material 10 umgeben. Das relativ feste Material 10 stellt eine gute Verbindung zum metallischen Kernmaterial 2 her und ist von der zweiten Schicht 11 umgeben. Die zweite Schicht 11 tritt im implantierten Zustand mit dem umgebenden Gewebe in Kontakt und weist die hierzu übliche weiche Beschaffenheit auf. Dies ermöglicht es, die Naht bei der Fixierung des Ringes durch das Innere rigide Gewebe 10 zu führen, um eine besonders gute Fixierung eines Klappenringes auch bei hohen punktuellen Belastungen zu gewährleisten.

Die Figur 5 zeigt, dass das Implantat 1 auch eine Fixierungslasche 12 aufweisen kann. Diese Fixierungslasche 12 wird im Ausführungsbeispiel von der zweiten Schicht 10 aus relativ festem Material gebildet. Durch die Ausbildung der Fixierungslasche 12 vergrößert sich der geometrische Bereich, in dem der Faden fixiert werden kann. Die Fixierungslasche 12 kann den gesamten Grundkörper ringförmig umgeben.

In Figur 6 ist ein Implantat mit zwei Fixationspunkten 13 und 14 gezeigt. Diese Fixationspunkte 13 und 14 liegen rechts und links von einem konkav gebogenen Randbereich des ringförmigen Grundkörpers. Diese Fixationspunkte sind so ausgeführt, dass hier zentral die Last in einen Klappenring eingekoppelt werden kann. Die Position der Fixationspunkte ist an die anatomischen Gegebenheiten angepasst und soll eine Fixierung an den Trigoni Fibrosae zulassen.

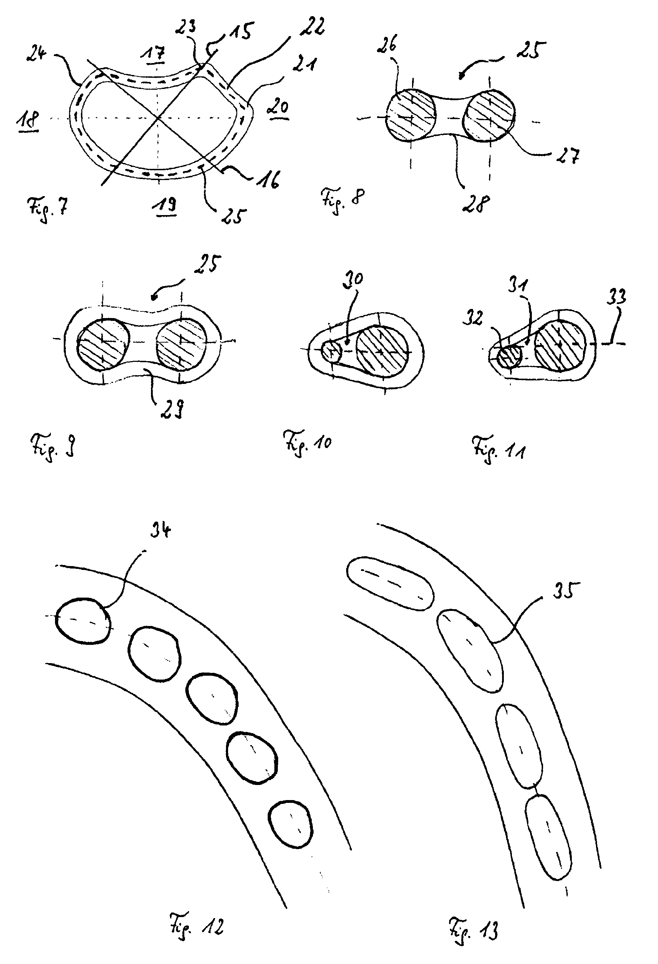

In Figur 7 ist ein Implantat mittels der orthogonal zueinander liegenden Linien 15 und 16 in vier Quadranten unterteilt. Im ersten Quadranten 17 ist die Randlinie des Implantats nach innen gebogen und somit konkav ausgebildet, während in den links daran anschließenden beiden weiteren Quadranten 18 und 19 die Randlinie des Implantats nach außen gebogen ist. Im vierten Quadranten 20 ist die Randlinie des Implantats zunächst wie in den davor liegenden Quadranten nach außen gebogen und etwa in der Mitte des vierten Quadranten ist ein Knick 21 vorgesehen, der zu einem abgeflachten oder leicht nach innen gebogenen Teilbereich 22 hinführt. Dieser Teilbereich 22 reicht bis zu einem weiteren Knick 23, der etwa beim Übergang vom vierten Quadranten zum ersten Quadranten liegt. Im Grundkörper 24 des Implantats sind längliche Stichkanäle 25 vorgesehen, von denen eine Vielzahl auf dem Grundkörper 24 des Implantats angeordnet sind.

Der in Figur 8 gezeigte Schnitt zeigt eine mögliche Ausführungsform derartiger Stichkanäle 25. Der Kanal 25 teilt den Grundkörper 24 im Bereich des Stichkanals in zwei im Querschnitt kreisförmige Grundkörperquerschnittsflächen 26, 27 auf. Durch die Ausbildung des Grundkörpers im Übergangsbereich zum Stichkanal entsteht eine trichterförmige Ausbildung 28, die es erleichtert, eine Nadel in den Stichkanal 25 einzuführen.

Die Stichkanäle ermöglichen eine Ausführungsform ohne ein den Grundkörper umgebendes Gewebe. Jedoch auch bei einer Verwendung eines den Grundkörper umgebenden Gewebes - wie in Figur 9 gezeigt - erleichtert der Kanal 25 die Verankerungen von Fäden am Grundkörper, sofern die Fäden durch das umgebende Gewebe 29 und den Stichkanal 25 geführt werden.

Die Figuren 10 und 11 zeigen, dass die Stichkanäle nicht in der Mitte des Grundkörpers angeordnet werden müssen. Ein leichtes Versetztes des Stichkanals 30 radial nach außen, wie in Figur 10 gezeigt, ermöglicht das Annähen des Implantats in seinem radial äußeren Bereich. In Figur 11 ist ein Stichkanal 31 gezeigt, bei dem der äußere Teil 32 des Grundkörpers versetzt zur vom Grundkörper umgebenden gedachten Fläche 33 angeordnet ist. Dies erleichtert das Annähen des Implantats am umliegenden Gewebe.

Auch bei trichterförmiger Ausbildung des Zugangs zum Stichkanal können die Stichkanäle wie in den Figuren 12 und 13 gezeigt, als runde Öffnungen 34 oder als ovale Öffnungen 35 ausgebildet sein. Die ovalen Öffnungen 35 erstrecken sich in Längsrichtung des Grundkörpers, um die Stabilität des Grundkörpers nur minimal zu beeinträchtigen.

Eine andere Beschreibung des Implantats sieht vor, dass der Grundkörper in der Draufsicht bohnenförmig mit einer abgeflachten Seite ausgebildet ist. Das dem Grundkörper umgrenzende, komplexe Zusammenspiel von konkaven und konvexen Linien entspricht im Wesentlichen der Außenkontur einer Bohne, wobei anschließend an die nach innengebogene Fläche eine abgeflachte oder eine weitere nach innen gebogene kürzere Fläche vorgesehen ist.

Die Ausführungsbeispiele zeigen Implantate, die für die Verwendung als Klappenring ausgebildet sind. Erfindungsgemäße Implantate können jedoch für unterschiedliche Einsatzzwecke in der Chirurgie und insbesondere zur Verstärkung von Auslässen und Gefäßen verwendet werden.

## Patentansprüche

1. Ringprothese für Anuloplastie (1) mit einem ringförmigen Grundkörper (2), ***dadurch gekennzeichnet, dass*** der Grundkörper (2) Stichkanäle aufweist.

2. Ringprothese für Anuloplastie (1) nach Anspruch 1, ***dadurch gekennzeichnet, dass*** die Stichkanäle trichterförmige Öffnungen aufweisen.

3. Ringprothese für Anuloplastie nach einem der vorhergehenden Ansprüche, ***dadurch gekennzeichnet, dass*** die Stichkanäle in der Draufsicht rund ausgebildet sind.

4. Ringprothese für Anuloplastie nach einem der vorhergehenden Ansprüche, ***dadurch gekennzeichnet, dass*** die Stichkanäle in der Draufsicht oval ausgebildet sind.

5. Ringprothese für Anuloplastie nach einem der vorhergehenden Ansprüche, ***dadurch gekennzeichnet, dass*** die Stichkanäle in der Mitte des Grundkörperquerschnitts angeordnet sind.

6. Ringprothese für Anuloplastie nach einem der vorhergehenden Ansprüche, ***dadurch gekennzeichnet, dass*** die Stichkanäle versetzt zur Mitte des Grundkörperquerschnitts vorzugsweise nach außen verlagert angeordnet sind.

7. Ringprothese für Anuloplastie nach einem der vorgehenden Ansprüche, ***dadurch gekennzeichnet, dass*** der Grundkörper (2) plastisch verformbar ist.

8. Ringprothese für Anuloplastie nach einem der vorhergehenden Ansprüche, ***dadurch gekennzeichnet, dass*** der Grundkörper (2) von einem mehrschichtigen Material, vorzugsweise Gewebe, umgeben ist.

9. Ringprothese für Anuloplastie nach einem der vorhergehenden Ansprüche, ***dadurch gekennzeichnet, dass*** der Grundkörper (2) von einem festen Material (10) umgeben ist und dieses von einem relativ weichen Material (11) umgeben ist.

10. Ringprothese für Anuloplastie nach einem der vorhergehenden Ansprüche, ***dadurch gekennzeichnet, dass*** der Grundkörper (2) ein metallisches Material aufweist.

11. Ringprothese für Anuloplastie insbesondere nach einem der vorhergehenden Ansprüche, ***dadurch gekennzeichnet, dass*** die Ringprothese für Anuloplastie (1) eine Fixierungslasche (12) aufweist.

12. Ringprothese für Anuloplastie nach Anspruch 11, ***dadurch gekennzeichnet, dass*** die Fixierungslasche (12) in einem den Grundkörper (2) umgebenen Material angeordnet ist.

13. Ringprothese für Anuloplastie nach einem der Ansprüche 11 oder *12, **dadurch gekennzeichnet, dass*** die Fixierungslasche (12) in einem den Grundkörper (2) umgebenen festen Material (10) ausgebildet ist.

14. Ringprothese für Anuloplastie nach einem der Ansprüche 11 bis 13, ***dadurch gekennzeichnet, dass*** die Fixierungslasche (12) den Grundkörper (2) ringförmig umgibt.

15. Ringprothese für Anuloplastie insbesondere nach einem der vorhergehenden Ansprüche 1 bis 14, ***dadurch gekennzeichnet, dass* die** Ringprothese für Anuloplastie mindestens einen verstärkt ausgebildeten Fixationspunkt (13, 14) aufweist.

16. Ringprothese für Anuloplastie nach Anspruch 15, ***dadurch gekennzeichnet, dass*** Fixationspunkte (13, 14) an zwei Seiten eines konkav gebogenen Randbereichs (8) angeordnet sind.
